# EUROPEAN PATENT SPECIFICATION

(11) **EP 2 515 782 B1**
(45) Date of publication and mention of the grant of the patent: **16.12.2015**
(21) Application number: 10842710.5
(22) Date of filing: 21.12.2010
(51) Int. Cl.: A61B 19/04, A41D 19/015, B32B 25/08, B32B 27/14

(54) **POWDER-FREE GLOVE WITH STABLE AND FAST-ACTING ANTIMICROBIAL COATING**
PULVERFREIER HANDSCHUH MIT STABILER UND SCHNELL WIRKENDER ANTIMIKROBIELLER BESCHICHTUNG
GANT SANS POUDRE AVEC REVÊTEMENT STABLE ET ANTIMICROBIEN À ACTION RAPIDE

(30) Priority: 21.12.2009 US 288420 P
(43) Date of publication of application: 31.10.2012
(73) Proprietor: Ansell Limited, Richmond, Victoria 3121 (AU)
(72) Inventor: ENG, Aik, Hwee, Petaling Jaya Selangor 47810 (MY); TANG, Lok, Si, Subang Jaya Selangor Darul Ehsan 47620 (MY); LUCAS, David, M., Petaling Jaya Selangor 47410 (MY); WAN AHMAD, Wan Ashruzi, Klang Selangor Darul Ehsan 41050 (MY)
(74) Representative: Zimmermann & Partner Patentanwälte mbB
(86) International application number: PCT/US2010/061481
(87) International publication number: WO 2011/084778

(56) References cited:
- WO-A2-2007/064344
- US-A1- 2005 002 995
- US-A1- 2005 112 180
- US-A1- 2006 070 167
- US-A1- 2007 020 342
- US-A1- 2007 077 348
- US-A1- 2007 104 766
- US-A1- 2007 104 766
- US-A1- 2009 139 012

## Description

This invention relates to elastomeric articles, specifically gloves that have one or more surfaces coated with an antimicrobial tack-free coating that kills germs fast.

Use of antimicrobial materials for use on gloves has been widely explored. Gloves that are coated with such antimicrobial materials generally have a large degree of tackiness, which leads to blocking of the gloves. As a result, donning and dispensing of such gloves are difficult. For example, when an antimicrobial-containing coating is on the interior surface of a glove, these surfaces tend to stick to each other preventing a user from easily inserting the hand into the glove. When the antimicrobial-containing coating is applied to the external surface of a glove, the gloves stick to each other preventing their extraction individually from a glove package or box.

U.S. Patent 4,853,978 to Stockum is directed to an antimicrobial medical glove. This antimicrobial medical glove consists of an elastomeric body in the shape of a hand and has an inner coating containing an antimicrobial agent. The inner coating contains corn starch with an antimicrobial agent and slowly releases the antimicrobial agent over a period of time sufficient to maintain an essentially bacteria-free and fungus-free environment within the donned glove. In Stockum, it is recognized that powders such as corn starch aids in antiblocking. This is not a powder free glove.

U.S. Patent Nos. 5,031,245; 5,180,605; and 5,261,421 to Milner disclose gloves, their manufacture and use. The antimicrobial natural rubber latex gloves may be manufactured by incorporating an antimicrobially effective amount of a non-ionic, sparingly water-soluble antimicrobial agent, such as 2,4,4'-tricloro-2'-hydroxyphenyl ether, into the glove material prior to forming the glove. The wearer contacting surface of the glove may also be dusted with a powder containing an antimicrobial effective amount of an antimicrobial agent such as chlorhexidine digluconate. The use of the powder on the glove surface does not produce a powder free glove.

U.S. Patent No. 5,089,205 to Huang discloses a process for producing medical devices having antimicrobial properties. The process involves partially forming the gloves by dipping glove molds into an anionically-stabilized natural latex composition and prior to curing or heating to final form, dipping into an anionically or non-ionically neutralized cationic antimicrobial agent and thereafter curing. This neutralization, however, likely reduces the effectiveness of the cationic antimicrobial agent and its long term microbe killing efficiency is unlikely. Alternatively, the antimicrobial composition can be additionally or independently applied to a glove (e.g., cured) before stripping. The result is a glove which, according to Huang, prevents, or decreases the potential of, cross-contamination between the glove users and patients because it will kill or reduce the susceptible microorganisms prior to or after penetration of the basic material forming the glove.

U.S. Patent No 5,888,441 to Milner discloses preparation of antimicrobial articles. This method for the manufacture of an antimicrobial rubber article includes a step of incorporating an effective amount of an antimicrobial agent into the natural rubber after the article has been shaped but before the article has been cured. The antimicrobial agent is chlorhexidine digluconate. Chlorhexidine digluconate forms a sticky film and the glove is not tack-free. The latex material may also include an antimicrobial agent such as 2,4,4'-trichloro-2'-hydroxy diphenyl ether (triclosan). The outer surface of the glove is not indicated to have an antimicrobial coating that is tack-free.

U.S. Patent No. 6,037,386 to Modak discloses composition for inactivating irritants in fluids. A topical composition containing zinc gluconate gel has an anti-irritant effect on the skin. The gel matrix may comprise chlorhexidine gluconate and the zinc gluconate gel diminishes the irritant and/or allergenic effect of the chlorhexidine gluconate. The combination of zinc gluconate and chlorhexidine gluconate will be sticky and the glove will not be tack free.

U.S. Patent Application No. 2003/0157150 to Lee discloses formulation and process for manufacturing antimicrobial vinyl gloves. In this manufacturing process, an effective amount of a powered antimicrobial agent is allowed to suspend in a polymer plastisol including a PVC resin, a plasticizer blend, a stabilizer, a surfactant and a dispersing agent. Then, a shape of the glove is dipped into a mixture of the polymer plastisol and powered antimicrobial agent. Then, the mixture on the shape is cured so as to form the glove. The glove does not have an antimicrobial coating, rather, the antimicrobial powder is incorporated into the PVC glove.

U.S. Patent Application No. 2003/0157152 to Hourihan et al. discloses an antimicrobial glove and method of making same. The antimicrobial glove comprises a glove material that is incorporated with diiodomethyl-p-tolylsulfone antimicrobial material that is homogeneously distributed throughout the glove material. The glove does not have a tack-free antimicrobial coating.

U.S. Patent Application No. 2005/0147655 to Bagwell discloses a non-leaching antimicrobial glove. The article has a coating of a hydrophobic, non-leaching antimicrobial polymer that is durably attached to an exterior surface, such that said antimicrobial polymer does not spontaneously migrate or is not removed from said exterior surface in the presence of aqueous substances, strong acids and bases, and organic solvents, and said antimicrobial polymer forms either a water-insoluble siloxane resin, or a covalently attached siloxane homopolymer, or a combination of both. The coated surface has a reduced affinity for aqueous-based substances and exhibits an enduring reduction in microbe affinity and transmission. The coating merely reduces the attachment of micro-organisms and does not kill microorganisms contacting the glove interior or exterior surface.

U.S. Patent Application No. 2007/0077348A1 to Lu et al. discloses a process for providing antimicrobial surfaces. The process for providing durable antimicrobial surfaces comprises treating a polymer substrate surface with formaldehyde in combination with a co-reactant followed by treatment with an antimicrobial peptide, which is a cationic linear peptide. The formaldehyde treatment allows the antimicrobial peptide to bond the polymer surface. The antimicrobial peptide is not a cationic antimicrobial composition.

European Patent Application EP1537796A2 to Chou discloses an antimicrobial elastomeric flexible article, such as a glove, and manufacturing method. The disposable protective glove comprises a first layer with an effective amount of antimicrobial agent 2,4,4'trichloro-2'-hydroxydiphenyl ether and soothing aloe vera gel. The antimicrobial glove coating is not indicated to be tack-free.

PCT Patent Application No. WO2007/058880A2 to Wang et al discloses a powder-free elastomeric article having a surface coating comprising an antimicrobial agent, hydrophilic film-forming polymer and a hydrophobic component. The antimicrobial agent in incorporated in a controlled-release matrix comprising a hydrophilic film-forming polymer and a hydrophobic component.. The hydrophilic polymer is believed to provide a reservoir for the antimicrobial agent, while the hydrophobic component is believed to improve the film's flexibility through its plasticizing effect. Paraffin wax is used for hydrophobicity enhancement, friction control, antiblocking and barrier properties. The antimicrobial activity of 2-log reduction is claimed against micro-organisms which is rather low for any practical application. A desiccant is needed in the packaging of the glove to maintain the stability of the glove.

PCT Patent Application No. WO99/52362 to Mixon discloses antimicrobial gloves and method of manufacturing thereof. Antimicrobial protection may be provided to protective gloves by mixing an antimicrobial agent in a glove material film so that the antimicrobial agent migrates to the exposed surfaces of the gloves when the agent on the glove surface has been depleted. The antimicrobial substance is dispersed entirely within the glove and the glove does not have an antimicrobial coating.

US 2007/0020342 A1 discloses a coating for application to an article, the coating comprising two water soluble zinc salts, each at a concentration of between 0.1 and 1 weight percent; a first water insoluble zinc salt at a concentration of between about 0.1 and 1 weight percent; a derivative of pantothenic acid at a concentration of between about 0.05 and 5 weight percent; and an effective amount of an antimicrobial agent such as inter alia biguanide.

US 2006/0070167 A1 discloses a hand-friendly rubber glove article comprising a dried coating of an emulsified hand-friendly mixture, which comprises at least one water-soluble humectant moisturizer, at least one water-insoluble occlusive moisturizer such as polydimethylsiloxane, at least one water-soluble lubricant, and at least one water-soluble surfactant.

There is a need for powder-free gloves having antimicrobial efficacy, where an antimicrobial-containing coating is durable, stable, and fast-acting, without compromising other performance properties such as donning and dispensing. Specifically, there is a need for a medical glove having antimicrobial properties that remain intact during long term storage.

Further, there is a need in the art for a polymeric glove article with an antimicrobial coating that is tack-free, can be applied to both the interior and exterior of the glove, and that has long-life antimicrobial activity with a killing effectiveness of dangerous germs typically in the range of 4 to 6 log reduction of microbial population. These and other objects and advantages, as well as additional inventive features, will be apparent from the detailed description provided herein.

### BRIEF SUMMARY

Provided are manufactured elastomeric, powder-free gloves with a non-tacky antimicrobial coating, as defined by the claims, which can be applied to one or both of the interior and exterior surfaces of the glove. Such gloves provide easy glove donnability on a hand or in a double glove donning situation. Also, these gloves can kill microbes present on the hand fast with a high microbial killing efficiency, typically in the range of 4 to 6-log reduction of microbial population within two minutes. The antimicrobial coating present on the external surface of the glove kills any microbes present on surfaces contacted by the glove external surface with a similar killing efficiency. Such gloves can be easily extracted from a boxed package one at a time due to lack of tackiness of the gloves. This microbe killing efficiency of the anti-microbial coating is preferably not diminished during shelf-life storage of the glove product.

In one or more embodiments, provided are gloves comprising a polymeric layer that is substantially free from defects, having a coating with an antimicrobial agent. The coating can be on one or both of the interior and exterior surfaces of the glove. These gloves can have a tack free highly effective antimicrobial surface. The coating comprises four components, which include an antimicrobial agent and three components that include a water-insoluble anti-tack or anti-stick agent, a water-soluble wetting agent, and a lubricant. In one embodiment, provided is an elastomeric medical glove having an antimicrobial surface coating comprising: a polymeric layer, and a dried, non-tacky coating on a surface of the polymeric layer, the coating comprising an antimicrobial agent, a wax agent, a lubricant, and a water-soluble wetting agent; wherein the glove comprises a residual powder of less than 2 mg per glove.

Methods are provided that comprise forming or providing a liquid-impervious polymeric glove; coating the polymeric glove with a non-tacky antimicrobial solution on one or both of the interior and exterior surfaces of the glove; and drying the solution to form a dried, non-tacky antimicrobial coating.

Provided, for example, is an elastomeric medical glove having an antimicrobial surface coating comprising: a polymeric layer, and a dried, non-tacky coating on a surface of the polymeric layer, the coating comprising an cationic antimicrobial agent, a wax agent, a lubricant, and a water-soluble wetting agent; wherein the glove comprises a residual powder of less than 2 mg per glove. Further provided, for example, is a method of reducing microbial load during medical procedures, the method comprising wearing the elastomeric medical glove, wherein the dried, non-tacky coating is on an inner surface of the glove.

Additionally provided, for example, is a method of making an elastomeric medical glove comprising: providing a polymeric glove, applying an aqueous solution to a surface of the glove, the aqueous solution comprising an antimicrobial agent, a wax agent, a lubricant, and a water-soluble wetting agent; and drying the aqueous solution to form a dried, non-tacky antimicrobial coating on the surface of the glove; wherein the glove comprises a residual powder of less than 2 mg per glove.

### BRIEF DESCRIPTION OF THE DRAWINGS

So that the manner in which the above recited features of the present invention can be understood in detail, a more particular description of the invention, briefly summarized above, may be had by reference to embodiments, some of which are illustrated in the appended drawings. It is to be noted, however, that the appended drawings illustrate only illustrative embodiments of this invention and are therefore not to be considered limiting of its scope, for the invention may admit to other equally effective embodiments.

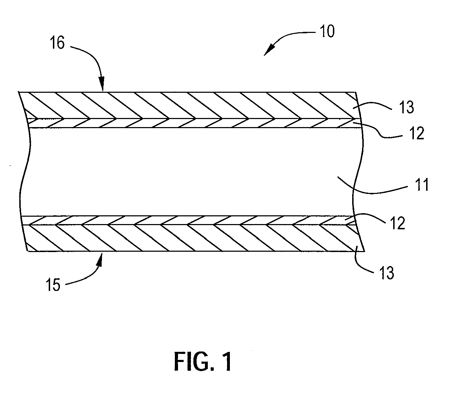

FIG. 1 illustrates a fragmentary cross-sectional view of a glove article according to an embodiment having a liquid impervious polymeric glove shell with a second polymeric surface followed by a non-tacky anti-microbial coating.

To facilitate understanding, identical reference numerals have been used, where possible, to designate comparable elements that are common to the figures. The figures are not drawn to scale and may be simplified for clarity. It is contemplated that elements and features of one embodiment may be beneficially incorporated in other embodiments without further recitation.

### DETAILED DESCRIPTION

Surgeons scrub their hands thoroughly prior to examination or surgery and donning of an examination or surgical glove. When an examination glove or surgical glove has an antimicrobial coating on the interior skin-contacting surface, any residual microbial organism present on the surgeon's hand is killed by the anti-microbial coating. If a glove is breached, the surgeon is protected from exposure to infectious organisms due to this antimicrobial coating. However, any contact of the previously sterilized donned glove with non-sterile surface such as a drape or gown results in the external surface of the glove being contaminated with microorganisms, which may be readily transferred to a patient. An antimicrobial coating present on the exterior surface of the glove prevents such microorganism transfer especially when the microbial kill rate of the antimicrobial coating is fast and is of a high value.

Provided is a powder-free glove with a stable, fast-acting antimicrobial coating on one or more surfaces. A "powder-free" glove has less than 2 mg per glove of powder, as measured by ASTM D6124-01 [ASTM International, West Conshohocken, PA, 2001, DOI: 10.1520/D6124-01]. The outside surface of the glove is non-tacky, and therefore does not adhere to other gloves when packed in bulk. This allows the glove to be removed from its pack without it sticking to another glove that is in contact with it. The outside surface can be non-slippery when in contact with water, providing a good grip to the user during use under wet conditions. The inside surfaces of the glove can be non-tacky, and therefore the interior of the glove does not stick to itself. This allows the user to open the cuff edge for donning without any issue. The antimicrobial property of the coating on one or more surfaces of the glove is indicated by the ability of the coating to reduce micro organisms, represented by gram positive bacteria, such as Staphylococcus aureus (ATCC 6538), and gram negative bacteria, such as Escherichia coli (ATCC 11229), by at least 4 log, or by 5 log, or by 6 log, when exposing a cut piece of 30 cm² of the glove's outside surface to the micro organisms for 2 minutes to an initial inoculum of 10⁸ colony forming units. The coating of the invention is antimicrobial if it induces the log 4 reduction against the above-named bacteria, given a 2 minute exposure to the bacteria. The effect on different test bacteria is typically measured separately. Such fast acting antimicrobial properties of the glove can remain intact (e.g., at ≥ 4 log, ≥ 5 log, ≥ 6 log) after the glove has been aged in an oven at 70°C for 10 days and/or 50°C for 90 days. These fasting acting antimicrobial properties of the glove can remain intact after the glove has been removed from the packaging and exposed to the air under ambient conditions over a period of 6 months, which is beneficial, for example, for unsterile exam gloves that are packed in bulk, e.g. 100 pieces per box, and may be exposed to the air after opening the packaging for weeks before being used.

The antimicrobial coating comprises a water soluble cationic antimicrobial agent comprising a biguanide. One such agent is poly(hexamethylene biguanide) chloride. Other antimicrobial biguanides are described in U.S. 6,559,116 and U.S. Patent 4,675,347. The amount in the coating composition can be for example, about 0.5% by weight or more, or about 3.0% by weight or less, or a range therebetween (such as from about any 1/10 percent point in the range to another 1/10 percent point in the range).

Among cationic antimicrobial biguanides are those of U.S. 4,675,347, which include: 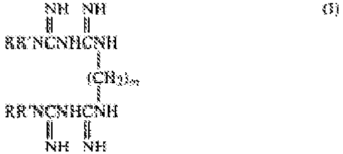 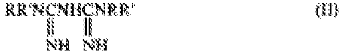 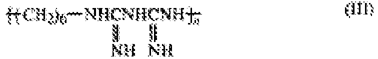 In the above formulae, R is an alkyl group, an aminoalkyl group, a phenyl group, an alkylphenyl group, a halophenyl group, a hydroxyphenyl group, a methoxyphenyl group, a carboxyphenyl group, a naphthyl group or a nitrile group; R' is a hydrogen atom or an alkyl group; and m and n each is a positive integer, such as an integer within the range of 2 to 10. Such biguanide compounds include 1,6- di (4-chlorophenylbiguanido)hexane, diaminohexylbiguanide, 1,6-di(aminohexylbiguanido)hexane and polyhexamethylenebiguanide.

As outlined in U.S. 6,559,116, exemplary biguanides include: chlorhexidine; 1,6-bis-(2-ethylhexylbiguanidohexane)dihydrochloride; 1,6-di-(N₁,N₁'-phenyldiguanido-N₅,N₅')-hexane tetrahydrochloride; 1,6-di-(N₁,N₁'-phenyl-N₁,N₁'-methyldiguanido-N₅,N₅')-hexane dihydro-chloride; 1,6-di(N₁,N₁'-o-chlorophenyldiguanido-N₅,N₅')-hexane dihydrochloride; 1,6-di(N₁,N₁'-2,6-dichlorophenyldiguanido-N₅,N₅')hexane dihydrochloride; 1,6-di[N₁,N₁'-.beta.-(p-methoxy-phenyl)diguanido-N₅,N₅']-hexane dihydrochloride; 1,6-di(N₁,N₁'-.alpha.-methyl-.beta.-phenyl-diguanido-N₅,N₅')-hexane dihydrochloride; 1,6-di(N₁,N₁'-p-nitrophenyldiguanido-N₅,N_{5'})hexane dihydrochloride;. omega.:. omega.'-di-(N₁,N₁'-phenyldiguanido-N₅,N₅')-di-n-propylether dihydro-chloride;. omega: omega'-di(N₁,N₁'-p-chlorophenyldiguanido-N₅,N₅')-di-n-propylether tetrahydro-chloride; 1,6-di(N₁,N₁'-2,4-dichlorophenyldiguanido-N₅,N₅')hexane tetrahydrochloride; 1,6-di(N₁,N₁'-p-methylphenyldiguanido-N₅,N₅')hexane dihydrochloride; 1,6-di(N₁,N₁'-2,4,5-trichlorophenyldiguanido-N₅,N₅')hexane tetrahydrochloride; 1,6-di[N₁,N₁'-.alpha.-(p-chloro-phenyl)ethyldiguanido-N₅,N₅']hexane dihydrochloride;. omega.:. omega.'di(N₁,N₁'-p-chloro-phenyldiguanido-N₅,N₅')m-xylene dihydrochloride; 1,12-di(N₁,N₁'-p-chlorophenyldiguanido-N₅,N₅')dodecane dihydrochloride; 1,10-di(N₁,N₁'-phenyldiguanido-N₅,N₅')-decane tetrahydro-chloride; 1,12-di(N₁,N₁'-phenyldiguanido-N₅,N₅')dodecane tetrahydrochloride; 1,6-di(N₁,N₁'-o-chlorophenyldiguanido-N₅,N₅') hexane dihydrochloride; 1,6-di(N₁,N₁'-p-chlorophenyldiguanido-N₅,N₅')-hexane tetrahydrochloride; ethylene bis(1-tolyl biguanide); ethylene bis(p-tolyl biguanide); ethylene bis(3,5-dimethylphenyl biguanide); ethylene bis(p-tert-amylphenyl biguanide); ethylene bis(nonylphenyl biguanide); ethylene bis(phenyl biguanide); ethylene bis(N-butylphenyl biguanide); ethylene bis(2,5-diethoxyphenyl biguanide); ethylene bis(2,4-dimethylphenyl biguanide); ethylene bis(o-diphenylbiguanide); ethylene bis(mixed amyl naphthyl biguanide); N-butyl ethylene bis(phenylbiguanide); trimethylene bis(o-tolyl biguanide); N-butyl trimethylene bis(phenyl biguanide); and the corresponding pharmaceutically acceptable salts of all of the above such as the acetates; gluconates; hydrochlorides; hydrobromides; citrates; bisulfites; fluorides; polymaleates; N-coconutalkylsarcosinates; phosphites; hypophosphites; perfluorooctanoates; silicates; sorbates; salicylates; maleates; tartrates; fumarates; ethylenediaminetetraacetates; iminodiacetates; cinnamates; thiocyanates; arginates; pyromellitates; tetracarboxybutyrates; benzoates; glutarates; monofluorophosphates; and perfluoropropionates, and mixtures thereof. Preferred antimicrobials from this group are 1,6-di-(N₁,N₁'-phenyldiguanido-N₅,N₅')-hexane tetrahydrochloride; 1,6-di(N₁,N₁'-o-chlorophenyl-diguanido-N₅,N₅')-hexane dihydrochloride; 1,6-di(N₁,N₁'-2,6-dichlorophenyldiguanido-N₅,N₅')hexane dihydrochloride; 1,6-di(N₁,N₁'-2,4-dichlorophenyldiguanido-N₅,N₅')hexane tetra-hydrochloride; 1,6-di[N₁,N₁'-.alpha.-(p-chlorophenyl) ethyldiguanido-N₅,N₅']hexane dihydro-chloride;.omega.:.omega.'di(N₁,N₁'-p-chlorophenyldiguanido-N₅,N₅')m-xylene dihydrochloride; 1,12-di(N₁,N₁'-p-chlorophenyldiguanido-N₅,N₅')dodecane dihydrochloride; 1,6-di(N₁,N₁'-o-chlorophenyldiguanido-N₅,N₅')hexane dihydrochloride; 1,6-di(N₁,N₁'-p-chlorophenyldiguanido-N₅,N₅')-hexane tetrahydrochloride; and mixtures thereof; more preferably, 1,6-di(N₁,N₁'-o-chlorophenyldiguanido-N₅,N₅')-hexane dihydrochloride; 1,6-di(N₁,N₁'-2,6-dichlorophenyl-diguanido-N₅,N₅')hexane dihydrochloride; 1,6-di(N₁,N₁'-2,4-dichlorophenyldiguanido-N₅,N₅')hexane tetrahydrochloride; 1,6-di[N₁,N₁'-.alpha.-(p-chlorophenyl)ethyldiguanido-N₅,N₅']hexane dihydrochloride;.omega.:.omega.'di(N₁,N₁'-p-chlorophenyldiguanido-N₅,N₅')m-xylene dihydrochloride; 1,12-di(N₁,N₁'-p-chlorophenyldiguanido-N₅,N₅')dodecane dihydro-chloride; 1,6-di(N₁,N₁'-o-chlorophenyldiguanido-N₅,N₅')hexane dihydrochloride; and 1,6-di(N₁,N₁'-p-chlorophenyldiguanido-N₅,N₅')-hexane tetrahydrochloride.

The cationic antimicrobial agents can be in the form of dermatologically acceptable salts. Mixtures of cationic antimicrobial agents can be used.

The antimicrobial coating comprises a water insoluble anti-tack or anti-stick polypropylene or polyethylene wax, such as a maleated polypropylene or polyethylene wax. The wax is typically provided as an emulsion (and may include emulsifying agents such as nonionic surfactant(s)). An exemplary polypropylene wax is Michem Emulsion 43040, having an average particle size of about 0.045 microns and a specific gravity in the range of 0.99 - 1.01. The amount in the coating composition can be for example, about 0.05, % by weight or more, or about 1.0% by weight or less, or a range therebetween (such as from about any 5/100 percent point in the range to another 5/100 percent point in the range). The wax emulsion can have for example an average particle size of about 0.1, 0.09, 0.08, 0.07, 0.06 or 0.05 microns or less.

The antimicrobial coating comprises a water soluble wetting agent. The wetting agent can be for example a cationic alkyl trimethyl ammonium bromide (such as Algene N40 from Huntsman Corporation, Woodlands, TX), or another water soluble wetting agent such as alkyl dimethyl benzyl bromide, oleyl bis (2-hydroxy ethyl) methyl ammonium chloride, or the like. Other dermatologically acceptable counter ions can be used in place of bromide. Exemplary alkyl trimethyl ammonium compounds include the dodecyl, myristyl and hexadecyl compounds. The amount in the coating composition can be for example, about 0.05% by weight or more, or about 1.0% by weight or less, or a range therebetween (such as from about any 5/100 percent point in the range to another 5/100 percent point in the range).

The antimicrobial coating comprises a water insoluble lubricant. The water insoluble lubricant comprises an aminofunctional siloxane. Exemplary aminofunctional siloxanes include, for example, that sold as Dow Corning DC939 (approximately 0.3 micron average particle size, a cationic emulsion of an aminofunctional silicone polymer containing a non-tallow surfactant, believed to contain Amodimethicone, Trideceth-12 (emulsifying agent, polyethylene glycol ether of tridecyl alcohol) and Octamethylcyclotetrasiloxane). (See Material Safety Data Sheet at http:/iwww4.dowcorning.com/DataFiles/090007b2815b86ad.pdf; Date viewed: 30 November 2010.) Other such amino-functional siloxanes include, for example, those described in US 4,127,872 The amount in the coating composition can be for example, about 0.05, 0.06, 0.07, 0.08, 0.09, 0.1, 0.1, 0.2, 0.3, 0.4 or 0.5% by weight or more, or about 1.0, 0.9, 0.8, 0.7, 0.6 or 0.5% by weight or less, or a range therebetween. In certain embodiments, the water insoluble lubricant is provided as an emulsion with an average particle size of about 0.03, 0.04, 0.05, 0.06, 0.07, 0.08, 0.09, 0.1, 0.1, 0.2, 0.3, 0.4 or 0.5 micron or more, or about 2, 1.9, 1.8, 1.7, 1.6, 1.5, 1.4, 1.3, 1.2, 1.1, 1.0, 0.9, 0.8, 0.7, 0.6 or 0.5 micron (micrometer) or less, or a range therebetween. The emulsion can include emulsifying agents such as nonionic surfactant(s).

The amino-functional siloxanes described in U.S. 4,127,872 include lubricants of the formula: 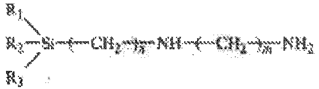 wherein R₁ is methyl or (CH₃)₃ SiO--, R₂ is methyl, R₃ is: 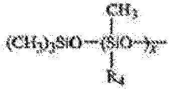 wherein R4 is methyl or --CH2)n NH--CH2)m NH2, n is an integer of at least 3, m is an integer of at least 2 and x can be 0 or an integer, and dimers thereof.

The antimicrobial agent is used to give stable durable antimicrobial properties to the glove. However, such agents are typically sticky and therefore can cause the glove's surfaces to stick to each other when applied alone onto the glove's surface. The anti-stick or anti-tack agent in combination with the lubricant is used to prevent the glove from sticking to each other after the coating and to provide good grip properties. The water-soluble wetting agent is used to reduce the surface tension of the antimicrobial mixture and help to spread the coating evenly on the glove's surface. All these four components comprising an antimicrobial agent, anti-tack agent, water soluble wetting agent and water insoluble lubricant are used to create a tack-free, powder-free antimicrobial glove coating that is evenly distributed on a glove surface.

Several cationic antimicrobial agents are available for incorporation into the antimicrobial coating. The resistances of these cationic antimicrobial agents to degradation vary due to differences in their chemical structure, molecular weight, and species of anions and anionic surfactants that tend to degrade the long lifetime of the antimicrobial coating. The cationic antimicrobial chlorhexidine gluconate (CHG) is recognized as a material that degrades as a function of time. However, poly(hexamethylene biguanide) chloride has been found to be more resistant to degradation than CHG. Mixtures of antimicrobial agents can be used, for example to expand the range of bacteria against which the coating is effective.

Gloves for use with the antimicrobial coating can be obtained as desired. That is, elastomeric gloves previously made can have the antimicrobial coating applied in the same manner as gloves that receive the antimicrobial in the production process. To make the glove having the antimicrobial coating in-process, the glove can first formed on a former using any suitable natural or synthetic lattices dipping method. Both powdered and powder free coagulant systems can be used. However, the powder free coagulant system is preferred as it will reduce the processing steps and time. The wet-gel glove that is formed after the dipping can then be leached in hot water, followed by for example chlorination or polymer coating to reduce the surface tackiness of the glove upon curing. The wet-gel glove can then cured (if appropriate) at elevated temperature using any suitable heating method to form the dry glove. The glove can then be optionally leached in hot water again to remove water-removable materials in the glove. The glove can then be optionally dipped in a silicone emulsion and dried to facilitate glove stripping. The outside surface of the glove becomes inside surface after stripping from the former. About 1,000 pieces, or 4 kg, of the glove are then processed as a batch in a tumbler dryer equipped with spray nozzles. The gloves are first heated to for example 60 to 70°C, followed by spraying with aqueous antimicrobial coating solution via attached nozzles. The antimicrobial solution. The quantity of antimicrobial solution sprayed is preferably in the range of 1-5 ml per glove, depending among other things on the surface area of the glove. Upon completion of spraying and drying, the gloves are unloaded from the dryer and ready for packing.

The polymeric material can be, for example, natural rubber, synthetic, such as polychloroprene, carboxylated acrylonitrile butadiene, styrene-butadiene, polyisoprene, polyurethane, polyvinyl chlorides, silicone rubbers, block copolymers (such as styrene-butadiene-styrene, styrene-isoprene-styrene, styrene-isoprene, styrene-butadiene or the like or combinations thereof) or the like or combinations thereof. The polymeric material can be a nitrile material, namely a co-polymerizate of butadiene and acrylonitrile, which can include co-polymerizates with minor amounts of modifying monomers, such as where acrylic or methacrylic acid are added to make a carboxylated nitrile rubber. Blends with compatible polymeric material are deemed nitrile rubber where the major portion by weight of the blend is a nitrile material. Other materials include for example styrene-butadiene-styrene or styrene-isoprene-styrene block copolymers, natural rubbers, or any combination of the polymeric materials discussed (given compatibility). The polymeric layers can be formed, for example, and as appropriate, by latex dipping, solvent dipping, or other appropriate methods. Polymeric layers can be heat cured as needed.

In certain embodiments, the coated gloves of the invention are dry, meaning not more than 2% w/w loss after drying in an appropriate drying apparatus at 105°C for about 2 hours. Similarly, a dried coating means that the coating and glove to which it is applied has not more than 2% w/w loss after drying in an appropriate drying apparatus at 105°C for about 2 hours.

To demonstrate the effectiveness of each material used in the antimicrobial coating, powder free nitrile examination gloves with different compositions of coating described in Table 1, were prepared according to the method described above and evaluated. The results are summarized in Table 2.

**Table 1 Composition of antimicrobial solution (balance is water)**

| Coating | Poly(hexamethylene biguanide) chloride, 2% | Polypropylene wax, 0.2%* | Alkyl trimethyl ammonium bromide, 0.2% | Amino functional siloxane, 0.2% |
|---|---|---|---|---|
| A | Yes | Yes | Yes | Yes |
| B | Yes | Yes | Yes | No |
| C | Yes | Yes | No | Yes |
| D | Yes | No | Yes | Yes |

| | | | | |
|---|---|---|---|---|
| *Particles size = 0.045micrometer, specific gravity = 0.99-1.01 Yes = present in the coating No = absent in the coating | | | | |

**Table 2 Properties of Antimicrobial gloves when packed in a box of 100 pieces**

| Coating | Dry Grip (outside) | Wet Grip (outside) | Stickiness (outside) | Stickiness (inside cuff) | Coating evenness (outside) | Residual powder, mg per glove |
|---|---|---|---|---|---|---|
| No coating | Good | Poor | Moderately Sticky | Non-sticky | - | < 2 |
| A | Good | Good | Non-sticky | Non-sticky | Even | < 2 |
| B | Good | Poor | Very sticky | Very sticky | Even | < 2 |
| C | Good | Good | Non-sticky | Non-sticky | Not even | < 2 |
| D | Good | Poor | Very sticky | Very sticky | Even | < 2 |

All gloves shown in the examples had a residual powder of less than 2 mg per glove, tested according to ASTM D 3577-06 (Specification for Rubber Surgical Gloves) and ASTM D 6124-01 (Standard Test Method for Residual Powder on Medical Gloves). From the results in Table 2, it is clear that the glove with Coating A gave the best combination of properties. As indicated by glove with coating B, a glove coating with polypropylene wax, in the absence of amino functional siloxane did not provide a non-tacky antimicrobial glove coating. Likewise, as indicated by glove with coating D, a glove coating with amino functional siloxane, in the absence of polypropylene wax was insufficient to provide a non-tacky antimicrobial glove coating.

To further test demonstrate the efficacy of the antimicrobial coating, a glove finger of 30 cm² area was cut from the fresh and aged glove with Coating A. The aging conditions employed were 70°C for 10 days, or 50°C for 90 days. They were tested against 2 micro-organisms at 2-minute exposure time. The initial inoculum is 10⁸ colonies forming units. Upon completion, the mixture containing the antimicrobial agent and micro organism was neutralized. Subsequently, the residual micro organism in the mixture was diluted in series, in the multiple of 10-fold. The micro organisms in the diluted mixtures were then grown in agar plates for 48 hours before counting the colony forming units. The results are summarized in Table 3.

**Table 3: The log reduction efficacy of antimicrobial glove with Coating A**

| | *Staphylococcus aureus* (ATCC 6538) | *Escherichia coli* (ATCC 11229), |
|---|---|---|
| Untreated glove | 1 | 1 |
| Unaged glove with Coating A | More than 6* | More than 6* |
| Aged glove with Coating A, 10 days at 70°C | More than 6* | More than 6* |
| Aged glove with Coating A, 90 days at 50°C | More than 6* | More than 6* |

| | | |
|---|---|---|
| *The micro organisms were completely killed in 2 minutes | | |

The results in Table 3 showed that the fast acting antimicrobial properties of an embodiment of a glove prepared using the present invention remained intact even after accelerated aging at 70°C for 10 days, or 50°C for 90 days. This implies that the antimicrobial properties can be expected to be stable throughout the shelf life of the glove.

Figure 1 at 10 depicts the manufactured glove article cross section with an anti-microbial coating. Should it be desirable, the coating can be placed in practice on either one or the other or both of the surfaces. A polymeric (e.g., liquid-impervious) shell of the glove is shown at 11 with an optional chlorinated or tackiness reducing polymeric coating 12 on the both the interior surface 15 and exterior surface 16 of the glove. The anti-microbial coating 13 is shown on both the interior surface 15 and exterior surface 16 of the glove.

Accordingly, in view of the above, in one or more embodiments, the method can comprise the steps of:
a) providing a liquid-impervious, polymeric shell (e.g. cured) produced by dipping a powdered or powder free coagulant-coated former into an aqueous polymeric material (such as a latex), coagulating a polymeric layer of wet gel glove on the former;
b) optionally leaching the wet glove in hot water, followed by optional chlorination or second polymer coating to reduce the surface tackiness ;
c) optionally curing wet-gel glove at elevated temperature using any suitable heating method to form dry glove;
d) optionally leaching in hot water again to remove water-removable materials in the glove;
e) optionally dipping in a silicone emulsion and drying to facilitate glove stripping;
f) heating said gloves (e.g., to 60 to 70°C, such as in a heated chamber) and spraying with the antimicrobial solution via the attached nozzles within a heated chamber;
g) drying the antimicrobial coated glove at a sufficient temperature for a sufficient period.

Where a pre-formed glove is treated, the glove is provided, and steps f) and g) can be conducted. Or, the method can comprise:
providing a polymeric glove (or forming the glove),
applying an aqueous solution to a surface of the glove, the aqueous solution comprising an antimicrobial agent, a wax agent, a lubricant, and a water-soluble wetting agent; and
drying the aqueous solution to form a dried, non-tacky antimicrobial coating on the surface of the glove;
wherein the glove comprises a residual powder of less than 2 mg per glove.

## Claims

1. An elastomeric medical glove having an antimicrobial surface coating comprising:
a polymeric layer, and
a dried, non-tacky coating on a surface of the polymeric layer, the coating comprising a cationic antimicrobial agent comprising a biguanide, a polyethylene or polypropylene wax, a lubricant comprising an aminofunctional siloxane, and a water-soluble wetting agent;
wherein the glove comprises a residual powder of less than 2 mg per glove.

2. The glove of claim 1, wherein the coating is formed from an aqueous solution of the antimicrobial agent in an amount in the range of 0.5 to 3.0% by weight, the polyethylene or polypropylene wax in an amount in the range of 0.05 to 1% by weight, the lubricant in an amount in the range of 0.05 to 1 % by weight, and the water-soluble wetting agent in an amount in the range of 0.05% to 1 % by weight.

3. The glove of claim 1, wherein the coating is formed from an aqueous solution of poly(hexamethylene biguanide) chloride in an amount in the range of 0.5 to 3.0% by weight, a polypropylene wax in an amount in the range of 0.05 to 1% by weight, an aminofunctional siloxane in an amount in the range of 0.05 to 1%, and alkyl trimethyl ammonium bromide in an amount in the range of 0.05% to 1%.

4. The glove of claim 1, wherein the polymeric layer comprises a polymeric material selected from the group consisting of natural rubber, synthetic rubber, and blends of natural and synthetic rubber.

5. The glove of claim 4, wherein the synthetic rubber is a nitrile rubber.

6. The glove of claim 4, wherein the synthetic rubber is polychloroprene, carboxylated acrylonitrile butadiene, styrene-butadiene, polyisoprene, polyurethane, polyvinyl chlorides, silicone rubber, styrene-butadiene-styrene block copolymer, styrene-isoprene-styrene block copolymer, styrene-isoprene block copolymer, styrene-butadiene block copolymer or a combination thereof.

7. The glove of claim 1, wherein the water-soluble wetting agent comprises alkyl trimethyl ammonium bromide.

8. The glove of claim 1, wherein the polymeric layer is a nitrile rubber and the coating comprises poly(hexamethylene biguanide) chloride (PHMB), a polypropylene wax, alkyl trimethyl ammonium bromide, and the aminofunctional siloxane.

9. The glove of claim 1, wherein the coating provides at least a 5 log reduction in Staphylococcus aureus (ATCC 6538) and Escherichia coli (ATCC 11229) after a 2 minute exposure.

10. The glove of claim 9 that has such an antimicrobial coating after storage for 10 days of accelerated aging at 70°C.

11. The glove of claim 9 that has such an antimicrobial coating after storage for 90 days of accelerated aging at 50°C.

12. A method of reducing microbial load during medical procedures, the method comprising wearing the elastomeric medical glove of claim 1, wherein the dried, non-tacky coating is on an inner surface of the glove.

13. The method of claim 12, wherein the coating provides at least a 5 log reduction in Staphylococcus aureus (ATCC 6538) and Escherichia coli (ATCC 11229) after a 2 minute exposure after storage for 10 days of accelerated aging at 70°C and after storage for 90 days at 50°C.

14. A method of making an elastomeric medical glove comprising:
providing a polymeric glove,
applying an aqueous solution to a surface of the glove, the aqueous solution comprising an antimicrobial agent comprising a biguanide, a polyethylene or polypropylene wax, a lubricant comprising an aminofunctional siloxane, and a water-soluble wetting agent; and
drying the aqueous solution to form a dried, non-tacky, fast-acting antimicrobial coating on the surface of the glove;
wherein the glove comprises a residual powder of less than 2 mg per glove.

## Patentansprüche

1. Elastomerer medizinischer Handschuh mit antimikrobieller Oberflächenbeschichtung, umfassend:
eine polymere Schicht und
eine getrocknete, nicht klebrige Beschichtung auf einer Oberfläche der polymeren Schicht, wobei die Beschichtung ein kationisches antimikrobielles Mittel umfassend ein Biguanid, ein Polyethylen- oder Polypropylenwachs, ein Gleitmittel umfassend ein aminofunktionelles Siloxan und ein wasserlösliches Netzmittel umfasst;
wobei der Handschuh einen Restpulvergehalt von weniger als 2 mg pro Handschuh umfasst.

2. Handschuh nach Anspruch 1, wobei die Beschichtung aus einer wässrigen Lösung des antimikrobiellen Mittels in einer Menge im Bereich von 0,5 bis 3,0 Gew.-%, des Polyethylen- oder Polypropylenwachses in einer Menge im Bereich von 0,05 bis 1 Gew.-%, des Gleitmittels in einer Menge im Bereich von 0,05 bis 1 Gew.-% und des wasserlöslichen Netzmittels in einer Menge im Bereich von 0,05 Gew.-% bis 1 Gew.-% gebildet ist.

3. Handschuh nach Anspruch 1, wobei die Beschichtung aus einer wässrigen Lösung von Poly(hexamethylen-biguanid)chlorid in einer Menge im Bereich von 0,5 bis 3,0 Gew.-%, eines Polypropylenwachses in einer Menge im Bereich von 0,05 bis 1 Gew.-%, eines aminofunktionellen Siloxans in einer Menge im Bereich von 0,05 bis 1% und von Alkyltrimethyl-ammoniumbromid in einer Menge im Bereich von 0,05% bis 1% gebildet ist.

4. Handschuh nach Anspruch 1, wobei die polymere Schicht ein polymeres Material, ausgewählt aus der Gruppe bestehend aus natürlichem Kautschuk, synthetischem Kautschuk und Mischungen aus natürlichem und synthetischem Kautschuk, umfasst.

5. Handschuh nach Anspruch 4, wobei es sich bei dem synthetischen Kautschuk um einen Nitrilkautschuk handelt.

6. Handschuh nach Anspruch 4, wobei es sich bei dem synthetischen Kautschuk um Polychloropren, carboxyliertes Acrylnitrilbutadien, Styrol-Butadien, Polyisopren, Polyurethan, Polyvinylchloride, Silicongummi, Styrol-Butadien-Styrol-Blockcopolymer, Styrol-Isopren-Styrol-Blockcopolymer, Styrol-Isopren-Blockcopolymer, Styrol-Butadien-Blockcopolymer oder eine Kombination davon handelt.

7. Handschuh nach Anspruch 1, wobei das wasserlösliche Netzmittel Alkyltrimethylammoniumbromid umfasst.

8. Handschuh nach Anspruch 1, wobei die polymere Schicht ein Nitrilkautschuk ist und die Beschichtung Poly(hexamethylenbiguanid)chlorid (PHMB), ein Polypropylenwachs, Alkyltrimethylammoniumbromid und das aminofunktionelle Siloxan umfasst.

9. Handschuh nach Anspruch 1, wobei die Beschichtung nach 2-minütigem Exponieren eine Reduktion an Staphylococcus aureus (ATCC 6538) und Escherichia coli (ATCC 11229) um mindestens 5 log bereitstellt.

10. Handschuh nach Anspruch 9, der solch eine antimikrobielle Beschichtung nach 10-tägiger Lagerung bei beschleunigter Alterung bei 70°C aufweist.

11. Handschuh nach Anspruch 9, der solch eine antimikrobielle Beschichtung nach 90-tägiger Lagerung bei beschleunigter Alterung bei 50°C aufweist.

12. Verfahren zum Reduzieren der Mikroorganismenbelastung während medizinischer Eingriffe, welches das Tragen des elastomeren medizinischen Handschuhs nach Anspruch 1 umfasst, wobei sich die getrocknete, nicht klebrige Beschichtung an einer Innenoberfläche des Handschuhs befindet.

13. Verfahren nach Anspruch 12, wobei die Beschichtung nach 10-tägiger Lagerung bei beschleunigter Alterung bei 70°C und nach 90-tägiger Lagerung bei 50°C nach 2-minütigem Exponieren eine Reduktion an Staphylococcus aureus (ATCC 6538) und Escherichia coli (ATCC 11229) um mindestens 5 log bereitstellt.

14. Verfahren zur Herstellung eines elastomeren medizinischen Handschuhs, das Folgendes umfasst:
Bereitstellen eines polymeren Handschuhs;
Auftragen einer wässrigen Lösung auf eine Oberfläche des Handschuhs, wobei die wässrige Lösung ein antimikrobielles Mittels umfassend ein Biguanid, ein Polyethylen- oder Polypropylenwachs, ein Gleitmittel umfassend ein aminofunktionelles Siloxan und ein wasserlösliches Netzmittel umfasst; und
Trocknen der wässrigen Lösung unter Bildung einer getrockneten, nicht klebrigen, rasch wirkenden antimikrobiellen Beschichtung an der Oberfläche des Handschuhs;
wobei der Handschuh einen Restpulvergehalt von weniger als 2 mg pro Handschuh umfasst.

## Revendications

1. Gant médical élastomère ayant un revêtement de surface antimicrobien comprenant :
une couche polymère et
un revêtement séché non poisseux sur une surface de la couche polymère, le revêtement comprenant un agent antimicrobien cationique comprenant un biguanide, une cire de polyéthylène ou de polypropylène, un lubrifiant comprenant un siloxane aminofonctionnel et un agent mouillant hydrosoluble ;
le gant comprenant une teneur de poudre résiduelle inférieure à 2 mg par gant.

2. Gant selon la revendication 1, dans lequel le revêtement est formé à partir d'une solution aqueuse de l'agent antimicrobien en une quantité dans la plage de 0,5 à 3,0 % en poids, de la cire de polyéthylène ou de polypropylène en une quantité dans la plage de 0,05 à 1 % en poids, du lubrifiant en une quantité dans la plage de 0,05 à 1 % en poids et de l'agent mouillant hydrosoluble en une quantité dans la plage de 0,05 % à 1 % en poids.

3. Gant selon la revendication 1, dans lequel le revêtement est formé à partir d'une solution aqueuse de chlorure de poly(hexaméthylènebiguanide) en une quantité dans la plage de 0,5 à 3,0 % en poids, d'une cire de polypropylène en une quantité dans la plage de 0,05 à 1 % en poids, d'un siloxane aminofonctionnel en une quantité dans la plage de 0,05 à 1 % et de bromure d'alkyltriméthylammonium en une quantité dans la plage de 0,05 % à 1 %.

4. Gant selon la revendication 1, dans lequel la couche polymère comprend un matériau polymère choisi dans le groupe constitué par le caoutchouc naturel, le caoutchouc synthétique et les mélanges de caoutchouc naturel et de caoutchouc synthétique.

5. Gant selon la revendication 4, dans lequel le caoutchouc synthétique est un caoutchouc nitrile.

6. Gant selon la revendication 4, dans lequel le caoutchouc synthétique est du polychloroprène, de l'acrylonitrile-butadiène carboxylé, du styrène-butadiène, du polyisoprène, du polyuréthane, des poly(chlorures de vinyle), du caoutchouc silicone, du copolymère séquencé de styrène-butadiène-styrène, du copolymère séquencé de styrène-isoprène-styrène, du copolymère séquencé de styrène-isoprène, du copolymère séquencé de styrène-butadiène ou une association de ceux-ci.

7. Gant selon la revendication 1, dans lequel l'agent mouillant hydrosoluble comprend du bromure d'alkyltriméthylammonium.

8. Gant selon la revendication 1, dans lequel la couche polymère est un caoutchouc nitrile et le revêtement comprend du chlorure de poly(hexaméthylènebiguanide) (PHMB), une cire de polypropylène, du bromure d'alkyltriméthylammonium et le siloxane aminofonctionnel.

9. Gant selon la revendication 1, dans lequel le revêtement assure au moins une réduction de 5 log de *Staphylococcus aureus* (ATCC 6538) et *d'Escherichia coli* (ATCC 11229) après une exposition de 2 minutes.

10. Gant selon la revendication 9 qui a un tel revêtement antimicrobien après stockage pendant 10 jours de vieillissement accéléré à 70 °C.

11. Gant selon la revendication 9 qui a un tel revêtement antimicrobien après stockage pendant 90 jours de vieillissement accéléré à 50 °C.

12. Procédé de réduction de charge microbienne pendant des actes médicaux, le procédé comprenant le fait de porter le gant médical élastomère selon la revendication 1, dans lequel le revêtement séché non poisseux est sur une surface interne du gant.

13. Procédé selon la revendication 12, dans lequel le revêtement assure au moins une réduction de 5 log de *Staphylococcus aureus* (ATCC 6538) et *d'Escherichia coli* (ATCC 11229) après une exposition de 2 minutes après stockage pendant 10 jours de vieillissement accéléré à 70 °C et après stockage pendant 90 jours à 50 °C.

14. Procédé de fabrication d'un gant médical élastomère comprenant :
la fourniture d'un gant polymère ;
l'application d'une solution aqueuse à une surface du gant, la solution aqueuse comprenant un agent antimicrobien comprenant un biguanide, une cire de polyéthylène ou de polypropylène, un lubrifiant comprenant un siloxane aminofonctionnel et un agent mouillant hydrosoluble ; et
le séchage de la solution aqueuse pour former un revêtement antimicrobien séché non poisseux à action rapide sur la surface du gant ;
dans lequel le gant comprend une teneur de poudre résiduelle inférieure à 2 mg par gant.
